# EUROPÄISCHE PATENTSCHRIFT

(11) **EP 3 356 115 B1**
(45) Veröffentlichungstag und Bekanntmachung des Hinweises auf die Patenterteilung: **11.11.2020**
(21) Anmeldenummer: 16766483.8
(22) Anmeldetag: 08.09.2016
(51) Int. Cl.: B29C 49/00, B29C 49/46, B29C 49/04

(54) **VERFAHREN ZUR REDUZIERUNG DER MIKROBIOLOGISCHEN BELASTUNG VON BEHÄLTERERZEUGNISSEN**
METHOD FOR REDUCING THE MICROBIOLOGICAL LOADING OF CONTAINER PRODUCTS
PROCÉDÉ POUR RÉDUIRE LA CHARGE MICROBIOLOGIQUE DE PRODUITS DU TYPE CONTENANT

(30) Priorität: 01.10.2015 DE 102015012939
(43) Veröffentlichungstag der Anmeldung: 08.08.2018
(73) Patentinhaber: Kocher-Plastik Maschinenbau GmbH, 74429 Sulzbach-Laufen (DE)
(72) Erfinder: GROH, Martin, 74405 Gaildorf (DE); BOHN, Christoph, 71566 Althütte (DE); SPALLEK, Michael, 55218 Ingelheim (DE)
(74) Vertreter: Bartels, Martin Erich Arthur
(86) Internationale Anmeldenummer: PCT/EP2016/001518
(87) Internationale Veröffentlichungsnummer: WO 2017/054904

(56) Entgegenhaltungen:
- DE-A1-102008 006 073
- US-A1- 2014 366 485
- US-A1- 2015 239 594

## Beschreibung

Die Erfindung betrifft ein Verfahren zur Reduzierung der mikrobiologischen Belastung von Behältererzeugnissen, die zumindest teilweise aus Kunststoffmaterial bestehen, bei dem im Rahmen einer ersten Herstellkette ein Kunststoffgranulat einer Extrudereinrichtung zugeführt wird, die das Granulat aufschmilzt, das im Rahmen einer weiteren, sich anschließenden Herstellkette an eine Blasform-, Füll- und Siegelherstellmaschine für den Erhalt des jeweiligen Behältererzeugnisses weitergeleitet wird.

Bei der Herstellung von Kunststoffbehältern einschließlich Ampullenerzeugnissen für Lebensmittel, Kosmetika oder für medizinische Zwecke, insbesondere Ophtalmika, Parenteralia oder für künstliche Ernährung, ist die mikrobiologische Qualität des Füllguts von wesentlicher Bedeutung. Die Anforderungen der internationalen Arzneibücher müssen erfüllt werden. Entscheidend hierfür ist zum einen die Sterilität des Füllguts vor der Befüllung, die z.B. durch Sterilfiltration erreicht werden kann. Zum anderen ist die Sterilität der Behälterinnenoberflächen von entscheidender Bedeutung.

Im Folgenden soll der Begriff "mikrobiologische Kontaminanten" als Zusammenfassung verstanden werden von Bakterien, Sporen, Hefen, Pilzen, Viren sowie Endotoxinen, die früher auch als Pyrogene bezeichnet wurden. Im Englischen spricht man fachsprachlich in diesem Zusammenhang auch von "Bioburden".

Zur Minimierung respektive weitgehenden Vermeidung mikrobiologischer Kontaminanten sind im Stand der Technik bereits Vorschläge gemacht worden. So beschreibt die DE 10 2008 032 635 A1 ein Verfahren für die Lebensmittel- und Getränkeindustrie zur mikrobiologisch optimierten Herstellung von blasgeformten Kunststoffbehältern. Dabei wird während des Blasvorgangs für den Kunststoffbehälter in das Innere des dahingehenden Vorformlings ein Medium, beispielsweise in Form von Luft, geleitet mit einer Temperatur zwischen 80 °C bis 140 °C, was im Sinne einer Sterilisierung der Abtötung von Keimen dienen soll. Damit dieses Verfahren wirken kann, sind im Hinblick auf die relativ niedrigen Behandlungstemperaturen sehr lange Behandlungszeiten notwendig, durchaus im Bereich von mehreren Stunden, um eine Abtötung von Keimen nachhaltig zu gewährleisten.

Die DE 10 2011 008 132 A1 beschreibt weiter ein Verfahren zur Herstellung von blasgeformten, mindestens bereichsweise sterilen Behältern, bei dem ein Vorformling aus einem thermoplastischen Material zunächst erwärmt und dann von einer Reckstange gereckt und mit einem unter Druck stehenden Fluid beaufschlagt wird, und ferner wird ein Sterilisationsmittel in den Bereich des Vorformlings zugeführt. Als Sterilisationsmittel wird bei dem bekannten Verfahren bevorzugt verdampftes Wasserstoffperoxid verwendet, das mit Heißluft gemischt wird, wobei die Wasserstoffperoxidkonzentration etwa 15 bis 35 Gewichtsprozent beträgt. Die Abbauprodukte dahingehender chemischer Sterilisationsmittel können das Füllgut verunreinigen und toxikologisch bedenkliche Folgen haben.

Durch die DE 695 20 445 T2 ist ein Verfahren zum sterilen Verpacken eines Getränks bekannt, bei dem im Rahmen des Blasformgebungsschrittes für den Behälter dieser auf eine Temperatur erhitzt wird, die ausreicht, um das Innere des Behälters zu sterilisieren. Da für eine sichere Sterilisation Temperaturen von deutlich über 200 °C viele Minuten lang notwendig sind, ist die Behältermaterialauswahl an Kunststoffen für dieses bekannte Verfahren entsprechend eingeschränkt und die bevorzugt für die Verpackung von Pharmazeutika verwendeten Polymere, wie Polyethylen oder Polypropylen, können dergestalt aufgrund ihrer niedrigen Einsatz- oder Schmelztemperaturen erst gar nicht verwendet werden.

Des Weiteren ist durch die DE 10 2008 006 073 A1 ein sog. Blasform-, Füll- und Siegel-Verfahren (BFS-Verfahren) bekannt, das sich in besonderer Weise für die Herstellung befüllter Behälter für medizinische Zwecke eignet. Hierzu gehören auch Ampullen als Behältererzeugnisse für Augentropfen mit Füllvolumina von beispielsweise 0,1 ml bis 10 ml wie auch Ampullen für Injektionslösungen im Bereich von typischerweise 0,5 ml bis 50 ml. Übliche Taktraten für die Herstellung dahingehend befüllter und verschlossener BFS-Behälter liegen im Bereich von 10 bis 18 Sekunden, bei modernen Anlagen, wie sie in der DE 10 2008 006 073 A1 aufgezeigt sind, beträgt die Taktzeit hingegen lediglich nur noch 2 bis 4 Sekunden. Bereits aufgrund dieser geringen Taktzeiten verbietet sich die Anwendung der oben genannten bekannten Sterilisationsverfahren, die so für BFS-Verfahren gar nicht einsetzbar sind, da sich an die Behälterformung die Befüllung innerhalb weniger Sekunden unmittelbar anschließt und ein Vorformling oder ein gar leerer Behälter für einen Sterilisationsvorgang erst gar nicht zur Verfügung steht.

Der mikrobiologische Status von Behältern, hergestellt nach dem BFS-Verfahren, wurde im Artikel von Frank Leo et al. "Evaluation of Blow-Fill-Seal-extrusion through processing Polymer Contaminated with bacterial Spores and Endotoxin", erschienen im PDA-Journal of Pharmaceutical Science and Technology, Bd. 58, Nr. 3, Mai-Juni 2004, Seiten 147-158, für den Sonderfall einer BFS-Anlage Typ 624 der Firma Weiler Engineering mit Taktraten von 12 bis 18 Sekunden (siehe Seite 148) beschrieben.

Hierbei wird in dem Fachartikel unter anderem aufgezeigt, dass es zur Reduzierung von Sporen durch zwei mögliche Mechanismen kommt. Zum einen die thermische Deaktivierung durch die lange Hitzeeinwirkung während der Herstellung (siehe Seite 153, unten links), zum anderen durch die erreichte homogene Verteilung (siehe Seite 153, 5. Absatz) der Sporen in der Schmelze und einer damit verbundenen möglichen Inaktivierung. Trotz dieser erzielten homogenen Verteilung berichten die Autoren jedoch nur von einer Keimzahlreduktion im Bereich von lediglich 10² bis 10⁴ keimbildenden Einheiten pro Gramm (KBE/g).

Die vorstehend bezeichneten Ergebnisse sind, was die Autoren ausdrücklich betonen, nicht auf andere Anlagen übertragbar, insbesondere nicht auf solche BFS-Anlagen mit deutlich geringeren Verweilzeiten bei erhöhter Temperatur, beispielsweise in Form von Anlagen der Firma rommelag vom Typ 460, die Gegenstand der technischen Lehre nach der DE 10 2008 006 073 A1 sind, bei denen die Taktraten, wie vorstehend angegeben, typischerweise im Bereich von weniger als 5 Sekunden liegen. Bei diesen Anlagen erfolgt kein Schneiden des warmen Polymerschlauchs und die Befüllung erfolgt durch sterile Füllrohre innerhalb des intakten plastifizierten Polymerschlauchs. Dergestalt stellt jedenfalls der Schlauch dann eine sterile Barriere gegenüber dem Außenraum dar.

Jedoch ist es leider nicht immer möglich, zu garantieren, dass das für den BFS-Prozess verwendete Polymergranulat mikrobiologisch ausreichend gering belastet ist. So kann es in der Praxis teilweise auch durch unsachgemäßen Transport, Lagerung und Handhabung des Kunststoffgranulats dazu kommen, dass mikrobiologische Kontaminanten, beispielsweise Sporen, auf die Granulatoberfläche gelangen können, was zu einer unerwünscht hohen mikrobiologischen Belastung führen kann, die durch das bisherige BFS-Verfahren nach dem Stand der Technik nicht immer ausreichend reduziert wird.

Ein steriles Verpackungsverfahren, das eine Gassterilisation einer BFS-Einrichtung vorsieht, geht aus der US 2015/0239594 A1 hervor.

Ausgehend von diesem Stand der Technik liegt der Erfindung die Aufgabe zugrunde, ein Verfahren bereit zu stellen, das sich bevorzugt im Rahmen des BFS-Herstellprozesses in diesen integrieren lässt und das die mikrobiologischen Kontaminanten signifikant reduzieren hilft.

Diese Aufgabe löst ein Verfahren mit den Merkmalen des Patentanspruchs 1 in seiner Gesamtheit.

Dadurch, dass gemäß dem kennzeichnenden Teil des Patentanspruchs 1 zumindest in Teilen der ersten Herstellkette das jeweils eingesetzte Kunststoffgranulat mindestens einem der folgenden Behandlungsschritte ausgesetzt wird:
- einer energiereichen Strahlung und/oder
- einer Plasmabehandlung und/oder
- einem sterilisierend wirkenden Gas, und
dass der jeweilige Behandlungsschritt erfolgt,
- wenn das Kunststoffgranulat noch zu sackartigen Gebinden zusammengefasst ist, nach deren Öffnen die Zuführung des Granulats in die Extrudereinrichtung im Rahmen der ersten Herstellkette erfolgt, oder
- wenn das Kunststoffgranulat im Rahmen einer Vereinzelung innerhalb der ersten Herstellkette im Rahmen einer flächigen schütt- oder trichterartigen Zuführung der Schnecke der Extrudereinrichtung zugeführt wird,
lässt sich mit jedem dieser Verfahren, respektive Behandlungsschritten, mit relativ geringem technischem Aufwand eine deutliche Reduzierung mikrobiologischer Kontaminanten innerhalb des Kunststoffgranulats, insbesondere an dessen Oberfläche, erreichen. Dies hat so keine Entsprechung im Stand der Technik.

Insbesondere, wenn das Polymergranulat mit energiereicher Strahlung behandelt wird, kommt es zu einer Reduzierung mikrobiologischer Kontaminanten an der Granulatoberfläche.

Ferner ist es möglich, zur Reduzierung von mikrobiologischen Kontaminanten eine Plasmabehandlung durchzuführen, beispielsweise unter Einsatz eines nicht thermischen, atmosphärischen Plasmas.

Ferner besteht die Möglichkeit durch chemische Sterilisation des Kunststoffgranulats die mikrobiologischen Kontaminanten zu reduzieren, was jedoch grundsätzlich die Gefahr von möglichen toxischen Rückständen im und am derart hergestellten Behältererzeugnis einschließlich Ampullen mit sich bringt.

Vorteilhaft bei der Verwendung von Verfahren mit energiereicher Strahlung ist es, dass die Durchstrahlung der Polymeren nicht notwendig ist. Eine üblicherweise eintretende Verfärbung von Polymeren durch energiereiche Strahlung ist praktisch nicht wahrnehmbar, da einerseits für die Behandlung der Granulatoberfläche nur geringe Strahlungsdosen notwendig sind und andererseits der unmittelbar nachfolgende thermische Extrusionsprozess im Rahmen des Blasform-, Füll- und Siegelverfahrens zur "Ausheilung" aktiver Farbzentren führt. So lassen sich die angesprochenen Bestrahlungsverfahren überraschenderweise auch anwenden auf an sich sehr strahlungsempfindliche Polymere, wie Polypropylen (PP), sowie nicht-transparente oder eingefärbte Polymere. Somit entfällt die bei den durchstrahlenden Verfahren aufwändige Anpassung der Bestrahlungswellenlänge an das jeweilige Behältermaterial.

Ferner ist es für einen Durchschnittsfachmann auf dem Gebiet der Herstellung von Kunststoff-Behältererzeugnissen überraschend, dass an sich nachteilige Kettenabbaureaktionen bei den angesprochenen Bestrahlungsverfahren nicht beobachtet werden oder sich jedenfalls nicht signifikant auf die Behälterqualität auswirken.

Weitere vorteilhafte Ausführungsformen des erfindungsgemäßen Verfahrens sind Gegenstand der Unteransprüche.

Im Folgenden wird das erfindungsgemäße Verfahren anhand einer Vorrichtung nach den Figuren näher erläutert. Dabei zeigen in prinzipieller und nicht maßstäblicher Darstellung die
- Fig. 1: eine stark vereinfacht gezeichnete Gesamt-Darstellung eines Ausführungsbeispiels einer Blasform-, Füll- und Siegelherstellmaschine gemäß der Lehre nach der DE 10 2008 006 073 A1 (Herstellmaschinen-Typ 460 der Firma rommelag);
- Fig. 2a: in stark vereinfacht gezeichneter Gesamt-Darstellung, teilweise im Längsschnitt gezeichnet, eine übliche Extrudereinrichtung mit eingangsseitigem Einfülltrichter für Kunststoffgranulat und ausgangsseitigem Schlauchkopf, der das geschmolzene Kunststoffmaterial eingangsseitig auf der Oberseite der BFS-Vorrichtung nach der Fig. 1 an diese abgibt; und
- Fig. 2b: stilisiert wiedergegeben ein sackartiges Gebinde mit eingefülltem Kunststoffgranulat für die Weiterverarbeitung mit Maschinenteilen gemäß den Darstellungen in Fig. 1 und 2a.

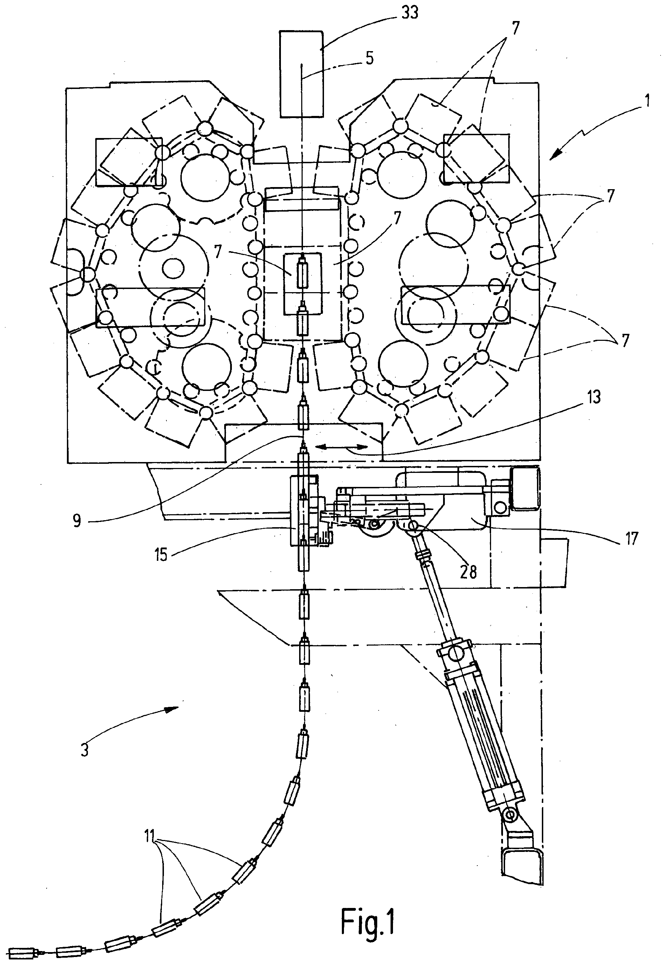

In Fig. 1 sind ein in der Figur obenliegender Herstellabschnitt als Ganzes mit 1 und eine sich nach unten daran anschließende Entformungseinrichtung als Ganzes mit 3 bezeichnet. Bei dem Herstellabschnitt 1 handelt es sich um eine Maschineneinrichtung zum Durchführen eines sog. Blasform-, Füll- und Siegelverfahrens entsprechend dem weitgehend bekannten bottelpack®-System, und zwar in einer Ausführungsform, bei der entlang einer Herstellungslinie 5 verschiedene Formgebungsschritte an verschiedenen Stationen durchgeführt werden. In einer Art Karussell-Anordnung werden hierbei Einzel-Formteile 7, von denen in der Fig. 1 lediglich einige beziffert sind, auf einer Art fiktiven Kreisbogenbahn paarweise aufeinander zu bewegt, um eine geschlossene Herstellform zu bilden, und zum Öffnen der Form wieder auseinander bewegt. Da nach dem bottelpack®-Verfahren arbeitende Maschineneinrichtungen an sich bekannt sind, erübrigt sich eine nähere Erläuterung der Einzelheiten des Herstellabschnitts 1 von Fig. 1.

Wie aus der Fig. 1 weiter ersichtlich ist, tritt die mittels der Einzel-Formteile 7 gebildete Behälterkette 9 entlang der Herstellungslinie 5 am unteren Ende des Herstellabschnitts 1 aus diesem hinaus und gelangt eingangsseitig zu der Entformungseinrichtung 3. Bei der Behälterkette 9 kann es sich um eine breitflächige Behälterkettenbahn handeln, bei der eine Mehrzahl von Einzelbehältern 11 ampullenartiger Gestalt als Kunststoff-Behältererzeugnisse in der Behälterkette 9 Seite an Seite nebeneinander liegend angeordnet sind.

Um das Ablösen der Behälter 11 von den Wänden der sich am Austrittsbereich voneinander weg bewegenden Einzel-Formteile 7 zu unterstützen, vermittelt die Entformungseinrichtung 3 der Behälterkette 9 eine Auslenkbewegung, wie sie in Fig. 1 mit Doppelpfeil 13 angedeutet ist. Zu diesem Zweck weist die Entformungseinrichtung 3 eine Mitnehmeranordnung 15 auf, die in getrieblicher Verbindung mit einem elektrischen Antriebsmotor 17 die Auslenkbewegung der Behälterkette 9 erzeugt, um die Behälter 11 von den Formwandteilen der Formteile 7 sicher abzulösen. Die näheren Einzelheiten zu dem weiteren Aufbau der dahingehenden Herstellmaschine 1 nebst Entformungseinrichtung 3 kann der DE 10 2008 006 073 A1 unmittelbar entnommen werden.

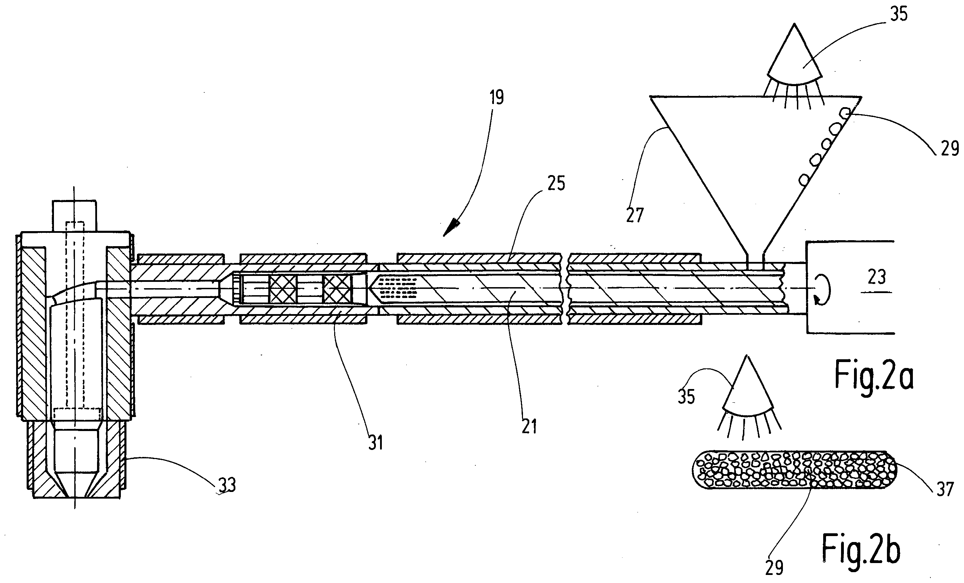

Die dahingehende Blasform-, Füll- und Siegelherstellmaschine 1 nebst Entformungseinrichtung 3 nach der Fig. 1 bildet im Rahmen des Gesamt-Herstellverfahrens eine weitere, sich anschließende Herstellkette zu einer ersten Herstellkette aus, die mit ihren wesentlichen Komponenten Gegenstand der Fig. 2a ist. Diese erste Herstellkette umfasst eine als Ganzes mit 19 bezeichnete Extrudereinrichtung unter Einsatz eines sog. Schneckenextruders, deren Förderschnecke 21 mittels eines Antriebs 23 antreibbar ist. Außenumfangsseitig sind an der drehbaren Förderschnecke 21 Heizeinrichtungen 25 angeordnet, um das mittels eines Zuführtrichters 27 oder einer sonstigen Schütteinrichtung zugeführte Kunststoffgranulat 29 zu plastifizieren, respektive zu schmelzen. Während sich der Zuführtrichter 27 auf der Eingangsseite der Förderschnecke 21 befindet, ist auf deren Ausgangsseite eine Mischereinrichtung 31 angeordnet, die das plastifizierte, respektive teilverflüssigte Kunststoffmaterial an einen Schlauchkopf 33 weiterleitet, der in vereinfachter Wiedergabe in Fig. 1 auf der Eingangsseite des Herstellabschnitts 1 eingezeichnet ist. Regelmäßig weist der dahingehende Schlauchkopf 33 einen Abgabequerschnitt auf, um dergestalt vorhangartig einen mantelseitig in sich geschlossenen Kunststoffschlauch an die Herstellmaschine 1 abzugeben. Der einfacheren Darstellung wegen wurden die weiteren Herstelleinrichtungen der Blasform-, Füll- und Siegelherstellmaschine 1 beispielsweise in Form der Fülldorne für das Einbringen des Behältermaterials ebenso weggelassen wie etwaige Blasdorne zum Erzeugen der Blasform sowie etwaig vorhandene Vakuumeinrichtungen zum verbesserten Anlegen des Kunststoffmaterials an die Forminnenwand der Einzel-Formteile 7. Die dahingehenden Herstellmaßnahmen sind bekannt, so dass an dieser Stelle hierauf nicht mehr näher eingegangen wird.

Die Verarbeitungstemperatur von Polyethylen als eingesetztem Kunststoffmaterial beträgt 170 °C bis 200 °C und bei Polypropylenmaterialien 190 °C bis 200 °C, wobei der Abgabedruck hinter der Extrudereinrichtung 19 an der Stelle des Übergangs zum Schlauchkopf 33 regelmäßig etwa 200 bar bis 400 bar beträgt.

Wie die Fig. 2a des Weiteren zeigt, besteht eine erfindungsgemäße Lösung darin, dass Kunststoff- oder Polymergranulat 29 mit energiereicher Strahlung unter Einsatz einer Strahlungsquelle 35 zu behandeln, die symbolhaft in die Zeichnung eingetragen ist. Die Strahlungsquelle 35 soll eine energiereiche Strahlung abgeben, um eine Reduzierung von mikrobiologischen Kontaminanten im vorstehend bezeichneten Sinne im Wesentlichen im Bereich der Granulatoberfläche zu erreichen. Dabei gelingt diese erfindungsgemäße Lösung vorteilhaft mit geringem Aufwand dadurch, dass man das Kunststoffoder Polymergranulat in der Zuführung zu der Extrudereinrichtung 19 beispielsweise innerhalb des Zuführtrichters 27 oder im Rahmen einer nicht näher dargestellten, aber üblichen Granulatrutsche flächig ausbreitet oder auffächert, wozu eine nicht näher dargestellte Labyrinthführung mit Hindernissen hilfreich sein kann, um dergestalt einer vorzugsweise flächigen Strahlungsquelle 35 die Bestrahlung des Granulats 29 auf mindestens einer, vorzugsweise mehreren Seiten zu ermöglichen.

Eine Möglichkeit der erfindungsgemäßen Granulat-Behandlung mittels der Strahlungsquelle 35 besteht im Aufbringen von Gammastrahlen, was jedoch einen entsprechend hohen Investitions- und Sicherheitsaufwand notwendig macht. Ungefährlicher ist das Aufbringen intensiver ultrakurzer Lichtblitze von sog. Xenon-Lampen mit einem hohen Anteil von UV-C-Strahlung, insbesondere im Wellenlängenbereich von 190 nm bis 290 nm mittels der Strahlungsquelle 35, was prinzipiell in der US 5 786 598 A beschrieben ist.

Ebenfalls möglich ist der Einsatz von monochromatischem UV-Licht von 193, 222, 248, 282, 308 und 354 nm Wellenlänge, wie dies prinzipiell in den Dokumenten US 2005/0173652 A1 und US 8 125 333 B2 (B. Ressler et al.) beschrieben ist. Ebenso möglich wäre das Aufbringen von Röntgenstrahlung über die Strahlungsquelle 35 gemäß den prinzipiellen Angaben in der Veröffentlichung WO 2008/129397 A8.

Als besonders vorteilhaft im Sinne der erfindungsgemäßen Lösung hat es sich jedoch erwiesen, Elektronenstrahlquellen einzusetzen, die oft auch als Betastrahler bezeichnet werden, mit relativ niedriger Elektronenstrahlenergie, da es ausreicht, die Granulatoberfläche mit nur einer geringen Eindringtiefe von wenigen Mikrometern in das eigentliche Kunststoffmaterial zu sterilisieren, wobei wiederum über die Strahlungsquelle 35 die Betastrahlen aufzubringen wären.

Die gewünschte Strahlendosis - typischerweise im Bereich von ca. 10 bis 25 kGray - kann leicht durch entsprechende Dimensionierung der Elektronenstrahlquelle 35 und der Verweilzeiten des Granulats 29 im Strahlbereich eingestellt werden. Vorzugsweis kommen dabei im Rahmen des fachsprachlich auch mit E-Beam bezeichneten Verfahrens kompakte Elektronenstrahlröhren mit 80 kV bis 300 kV-Beschleunigungsspannung zum Einsatz, womit man regelmäßig eine Eindringtiefe bis ca. 300*µ*m in den typischerweise im BFS-Prozess eingesetzten Polymermaterialien erzielen kann. Bei einer 300 mm Bestrahlungslänge auf einer Granulatrutsche (nicht dargestellt) wurden 25 kGray dabei schon bei 120 kV bis 150 kV-Beschleunigungsspannung erreicht. Bekannte, nachteilige Kettenabbaureaktionen wurden überraschenderweise selbst bei Polypropylen bei Einsatz der jeweiligen Strahlung über die Strahlungsquelle 35 nicht beobachtet oder wirkten sich jedenfalls nicht signifikant auf die Behälterqualität der Einzelbehälter 11 der Behälterkette 9 aus.

Zusätzlich oder alternativ zu der genannten Bestrahlung ist ebenfalls eine Plasmabehandlung möglich, wobei vorzugsweise ein nicht-thermisches, atmosphärisches Plasma verwendet wird, wie es beispielsweise beschrieben ist im Artikel von Tobias G. Klämpfl et al. "Cold Atmospheric Air Plasma Sterilization against Spores and Other Microorganisms of Clinical Interest", erschienen in der Zeitschrift Applied and Environmental Microbiology, Bd. 78, August 2012, Nr. 15, S. 5077-5082. Zusätzlich zu der Strahlungsquelle 35 oder an die Stelle derselben tritt dann eine nicht näher dargestellte Auftrageinrichtung für das zu erstellende atmosphärische Plasma, das in den Zuführtrichter 27 oder eine sonstige Zuführeinrichtung für die Extrudereinrichtung 19 ein- oder aufzubringen ist. Sollte nur ein Plasma-Auftragsprozess stattfinden, kann die Strahlungsquelle 35 also insoweit dann entfallen.

Ferner besteht die Möglichkeit, durch chemische Sterilisation des Granulats die mikrobiologischen Kontaminanten zu reduzieren. Hierfür kommen Gase, wie Ozon, Wasserstoffperoxid, Ethylenoxid, Stickstoffdioxid und andere Gase zum Einsatz, wie sie beispielsweise in der CDC (Center for Desease Control and Prevention) Guideline for Desinfection and Sterilization in Healthcase Facilities, 2008, von William A. Rutala et al. beschrieben sind. Für das Einbringen der dahingehenden Gase ist wiederum in den Bereich des Zuführtrichters 27 oder einer sonstigen Eintrageinrichtung für das Granulat 29 eine Ein- oder Auftragvorrichtung (nicht näher dargestellt) hierfür vorzusehen.

Wie die Darstellung nach der Fig. 2b zeigt, kann dem Grunde nach das jeweilige erfindungsgemäße Verfahren auch auf sackartige Gebinde 37 mit Kunststoffgranulat 29 Anwendung finden, wobei im Rahmen der Bestrahlungsverfahren - insbesondere bei Einsatz von Gamma- oder Röntgenstrahlen - das sackartige Gebilde 37 auch geschlossen bleiben kann, bevor dann nach der Bestrahlung über die externe Strahlungsquelle 35 und Öffnen des Gebindes 37 die Zuschüttung des Granulats 29 über die Schütt-Einrichtung 27 in die Extrudereinrichtung 19 erfolgt.

Im Rahmen einer praktischen Überprüfung der erfindungsgemäßen Lösung wurden für alle Ausführungsbeispiele Materialien, Behältergrößen und Maschineneinstellungen gewählt, die den ungünstigsten Fall (worst case) betreffend den Mechanismus der Reduzierung mikrobiologischer Kontaminanten darstellen. Als Beispiel für mikrobiologische Kontaminanten wurden, wie in der Sterilitätsprüfung üblich, resistente Sporen von Bazillus atrophaeus und Bazillus pumilus als Testkeime ausgewählt. Als Behältermaterialien wurden ferner Polymere verwendet, die niedrige BFS-Verarbeitungstemperaturen aufweisen, um thermische Effekte auf die künstlich zugesetzten Sporen gering zu halten. Auch wurden Prozessparameter gewählt, die möglichst nur minimale Effekte auf die Sporen ausüben, jedoch zu Behältererzeugnissen in verwendbarer Qualität und üblichen Ausbeutemengen führen. So wurde der Durchsatz von Polymer durch den Extruder 19 an der oberen Grenze eingestellt, um damit die Dauer der Wärmeeinwirkung auf die künstlich zugesetzten Sporen zu minimieren.

Ferner wurde eine BFS-Anlage vom Typ 460 der Firma rommelag, Waiblingen, Deutschland, verwendet, mit einer Zykluszeit für die Behälterherstellung von ca. 3,5 Sekunden. Als Kunststoffgranulat 29 wurden Polymere eingesetzt wie Pureil LDPE Type 1840 H der Firma LyondellBasell sowie Ineos LDPE Type Eltex MED PH 23H630 mit Verarbeitungstemperaturen des Extruders 19 wie auch des Schlauchkopfes 33 im Bereich zwischen 160 °C bzw. 165 °C.

Zur Herstellung der kontaminierten Granulat-Proben wurden Endosporen des Bazillus atrophaeus ATTC 9372 mit einem D-Wert, D_{160 °C} = 0,285 ± 0,08 min verwendet. In analoger Weise wurden Sporen des Referenzkeims für die Strahlensterilisation, Bazillus pumilus ATCC 27142, eingesetzt. Die Sporen wurden gleichmäßig auf das Granulat 29 verteilt und der Sporengehalt labortechnisch verifiziert. Der Konzentrationsbereich betrug 10³ bis 10⁶ KBE pro Gramm. Es wurden mit 6 ml flüssiger CASO-Nährlösung befüllte Behälter 11 der Größen 10 ml hergestellt.

Zur näheren Erläuterung: CASO-Nährlösung ist ein Komplexmedium, dem, außer Glucose, aus Milcheiweiß proteolytisch gewonnenes Pepton (Caseinpepton) und aus Sojamehl proteolytisch gewonnenes Pepton (Sojamehlpepton) zugesetzt wird. Das Caseinpepton ist reich an freien Aminosäuren und das Sojamehlpepton zeichnet sich durch einen hohen Gehalt an Kohlenhydraten und Vitaminen aus. Solche Nährmedien eignen sich besonders zur Kultivierung von anspruchsvollen Mikroorganismen.

Je Testcharge wurden über 12.000 Behältererzeugnisse gefertigt, wobei ansonsten die analytische Vorgehensweise dem Inhalt des eingangs erwähnten Artikels von Frank Leo et al. "Evaluation of Blow-Fill-Seal-extrusion through processing Polymer Contaminated with bacterial Spores and Endotoxin" entspricht.

Zunächst wurden drei Referenzchargen, d.h. ohne Anwendung der erfindungsgemäßen Methoden, zur Keimzahlreduzierung gefertigt. Hierfür wurde ein in der Extrusionstechnik üblicher Steckdornverteiler mit Löchern als dynamische Mischereinrichtung (wie im Buch von W. Michaeli gezeigt) eingesetzt, wobei eine erfindungsgemäße Granulat-Behandlung nicht stattfand. Hierbei ergab sich eine Keimzahlreduktion insbesondere aufgrund thermischer Effekte von durchschnittlich 10³ KBE/Gramm.

In einem der Versuche wurde dann das mit Sporen des Bazillus pumilus kontaminierte Polymergranulat 29 über eine vibrierende Rutsche, vergleichbar dem Zuführtrichter 27, der Extrudereinrichtung 19 zugeführt und mit einer Elektronenstrahlquelle 35 (E-beam) der Firma Comet AG, Schweiz, berührungslos, aber in nur sehr geringem Abstand mit unterschiedlichen Strahlungsdosen von ca. 10, 15 bzw. 25 bzw. 30 kGray bestrahlt. Bei 300 mm Bestrahlungslänge wurden 25 kGray bei 140 kV Beschleunigungsspannung erreicht, das dabei entstehende Ozon wurde abgesaugt; der Beitrag des Ozons zur Keimabtötung wurde nicht quantifiziert, ist aber generell nicht unerwünscht. Sollte die Ozonbildung aus anderen Gründen unerwünscht sein, kann dies leicht erreicht werden indem Luft durch eine Schutzgasatmosphäre aus beispielsweise Stickstoff, Argon oder Kohlendioxid ersetzt wird.

Bei diesen Versuchen hat sich gezeigt, dass eine rotierende Bewegung der Granulatkörner des Kunststoffgranulats 29 und eine flächige Auffächerung des Granulats 29 vorteilhaft ist, um möglichst Abschattungen zu vermeiden und so die komplette Oberfläche der Granulatkörner der energiereichen Strahlung mittels der jeweiligen Strahlungsquelle 35 auszusetzen.

Ferner wurden Versuche durchgeführt unter Einsatz der vorstehend näher bezeichneten Elektronenstrahlquelle 35, wobei Sporen des Bazillus atrophaeus das Polymergranulat 29 kontaminierten.

Das jeweilige Bestrahlungsverfahren hat einen Verbesserungsfaktor gegenüber der vorstehend bezeichneten Referenzmessung um mindestens den Faktor 1000 ergeben, d.h. die 1000-fache Menge an biologischen Kontaminanten konnte abgetötet werden.

Alle vorstehend genannten erfindungsgemäßen Methoden zur Minimierung der mikrobiologischen Belastung haben den Vorteil, dass nicht ein leerer Kunststoffbehälter oder gar ein bereits befüllter Behälter sterilisiert werden muss, sondern lediglich das noch feste Granulat vor dem Aufschmelzen.

Bei der Herstellung von Mehrschichtbehältern nach dem BFS-Verfahren, wie z.B. in der DE 103 47 908 A1 aufgezeigt, genügt gegebenenfalls die Anwendung der jeweils bezeichneten, erfindungsgemäßen Reduziermethode lediglich für das Polymergranulat, das die Innenschicht des Behälters 11 bildet.

Ein Verfahren zur Reduzierung der mikrobiologischen Belastung von Kunststoffbehältererzeugnissen, hergestellt nach dem Blasform-, Füll- und Siegel-verfahren, bei dem der Kunststoff vor dem Austritt aus dem Schlauchkopf 33 einem Behandlungsschritt mit sterilisierend wirkenden Gasen, energiereicher Strahlung und/oder einer Plasmabehandlung ausgesetzt ist, ist so im Stand der Technik nicht beschrieben.

## Patentansprüche

1. Verfahren zur Reduzierung der mikrobiologischen Belastung von Behältererzeugnissen (11), die zumindest teilweise aus mindestens einem Kunststoffmaterial bestehen, bei denen im Rahmen einer ersten Herstellkette ein Kunststoffgranulat (29) einer Extrudereinrichtung (19) zugeführt wird, die das Granulat (29) aufschmilzt, das im Rahmen einer weiteren, sich anschließenden Herstellkette an eine Blasform-, Füll- und Siegelherstellmaschine (1) für den Erhalt des jeweiligen Behältererzeugnisses (11) weitergeleitet wird, wobei zumindest in Teilen der ersten Herstellkette das jeweils eingesetzte Kunststoffmaterial mindestens einem der folgenden Behandlungsschritte ausgesetzt wird:
- einer energiereichen Strahlung und/oder
- einer Plasmabehandlung und/oder
- einem sterilisierend wirkenden Gas, und **dadurch gekennzeichnet dass** der jeweilige Behandlungsschritt erfolgt,
- wenn das Kunststoffgranulat (29) noch zu sackartigen Gebinden (37) zusammengefasst ist, nach deren Öffnen die Zuführung des Granulats (29) in die Extrudereinrichtung (19) im Rahmen der ersten Herstellkette erfolgt, oder
- wenn das Kunststoffgranulat (29) im Rahmen einer Vereinzelung innerhalb der ersten Herstellkette im Rahmen einer flächigen schütt- oder trichterartigen Zuführung (27) der Schnecke (21) der Extrudereinrichtung (19) zugeführt wird.

2. Verfahren nach Anspruch 1, **dadurch gekennzeichnet, dass** die energiereiche Strahlung als
- Betastrahlung,
- Gammastrahlung,
- UV-Strahlung,
- monochromatische UV-Strahlung,
- Röntgenstrahlung, oder
- Folge kurzer Lichtblitze
auf das insbesondere granulatförmige Kunststoffmaterial aufgebracht wird.

3. Verfahren nach Anspruch 1 oder 2, **dadurch gekennzeichnet, dass** für die Plasmabehandlung ein
- atmosphärisches Plasma
für das insbesondere granulatförmige Kunststoffmaterial eingesetzt wird.

4. Verfahren nach einem der vorstehenden Ansprüche, **dadurch gekennzeichnet, dass** als sterilisierend wirkendes Gas
- Ozon,
- Wasserstoffperoxid,
- Ethylenoxid oder
- Stickstoffdioxid
für das insbesondere granulatförmige Kunststoffmaterial eingesetzt wird.

5. Verfahren nach einem der vorstehenden Ansprüche, **dadurch gekennzeichnet, dass** der jeweilige Behandlungsschritt derart erfolgt, dass das durch Bewegen vereinzelte Kunststoffgranulat (29) zumindest entlang eines Teils seiner Oberfläche mit einer geringen Eindringtiefe von vorzugsweise wenigen Mikrometern behandelt wird.

6. Verfahren nach einem der vorstehenden Ansprüche, **dadurch gekennzeichnet, dass** als zu behandelnde Kunststoffmaterialien Thermoplaste, insbesondere Polymere, wie PE, HDPE, PP, PET, COC, COP, EVOH und PA zum Einsatz kommen, vorzugsweise gut extrudierbare Werkstoffe, wie Low Density Polyethylen (LDPE), PP oder HDPE.

7. Verfahren nach einem der vorstehenden Ansprüche, **dadurch gekennzeichnet, dass** die einzelnen Herstellschritte steril zu Herstell-Taktzeiten für ein keimarmes, befülltes und verschlossenes Behältererzeugnis (11) von 2 bis 4 Sekunden führen.

8. Verfahren nach einem der vorstehenden Ansprüche, **dadurch gekennzeichnet, dass** mittels der Extrudereinrichtung (19) ein Co-Extrusionsverfahren durchgeführt wird, was zu einem Mehrschichtaufbau der Wand des geschlossenen und befüllten Behältererzeugnisses (11) führt und, dass nur das die Innenwand des derart co-extrudierten Behälters (11) bildende Polymer einem der vorstehenden Behandlungsschritte zur Reduzierung von biologischen Kontaminanten unterzogen wird.

## Claims

1. Method for reducing the microbiological loading of container products (11) which at least partially consist of at least one plastics material, in which plastic granules (29) are supplied to an extruder device (19) as part of a first production process, said extruder device melting the granules (29), which are passed on to a blow-fill-seal manufacturing machine (1) to obtain the respective container product (11) as part of a further, adjoining production process, the plastics material used in each case being subjected to at least one of the following treatment steps at least in parts of the production process:
- high-energy radiation and/or
- plasma treatment and/or
- a gas with a sterilising effect,
and **characterised in that** the respective treatment step takes place
- if the plastic granules (29) are still packed together in sack-like packages (37) and, when said packages are opened, the granules (29) are fed into the extruder device (19) as part of the first production process or
- if the plastic granules (29) are supplied as part of a flat bulk or funnel-like feed (27) to the screw conveyor (21) of the extruder device (19) as part of a separation process within the first production process.

2. Method according to claim 1, **characterised in that** the high-energy radiation is applied to the plastics material, particularly in granule form, as
- beta radiation,
- gamma radiation,
- UV radiation,
- monochromatic UV radiation,
- X-ray radiation or
- a sequence of short flashes of light.

3. Method according to either claim 1 or claim 2, **characterised in that** an
- atmospheric plasma
is used for plasma treatment of the plastics material, particularly in granule form.

4. Method according to any one of the preceding claims, **characterised in that**
- ozone,
- hydrogen peroxide,
- ethylene oxide or
- nitrogen dioxide
is used as a gas with sterilising effect for the plastics material, particularly in granule form.

5. Method according to any one of the preceding claims, **characterised in that** the respective treatment step takes place in such a way that the plastic granules (29), which are separated by movement, are treated at least along part of their surface with a small penetration depth of preferably a few microns.

6. Method according to any one of the preceding claims, **characterised in that** thermoplastics, especially polymers such as PE, HDPE, PP, PET, COC, COP, EVOH and PA, preferably materials that are easy to extrude, such as low-density polyethylene (LDPE), PP or HDPE, are used as the plastics materials to be treated.

7. Method according to any one of the preceding claims, **characterised in that** the individual production steps lead in a sterile manner to production intervals of 2 to 4 seconds for a filled and sealed container product with low microbiological contamination (11).

8. Method according to any one of the preceding claims, **characterised in that** a co-extrusion method is performed using the extruder device (19), which leads to the wall of the sealed and filled container product (11) having a multi-layer construction, and **in that** only the polymer forming the inner wall of the container (11) co-extruded in this manner is subjected to one of the above-mentioned treatment steps to reduce biological contaminants.

## Revendications

1. Procédé de réduction de la charge microbiologique de produits (11) de type contenant, constitués, au moins en partie, au moins d'une matière plastique, dans lequel, dans le cadre d'une première chaîne de fabrication, on envoie un granulé (29) de matière plastique à un dispositif (19) d'extrudeuse, qui fond le granulé (29) que, dans le cadre d'une autre chaîne de fabrication qui s'y raccorde, on achemine à une machine (1) de fabrication par moulage, par soufflage, par remplissage et par scellement, afin d'obtenir le produit (11) du type contenant respectif, dans lequel au moins dans des parties de la première chaîne de fabrication, la matière plastique utilisée est soumise au moins à l'un des stades de traitement suivants :
- un rayonnement riche en énergie et/ou
- un traitement au plasma et/ou
- un gaz à effet de stérilisation, et **caractérisé en ce que** le stade de traitement s'effectue,
- lorsque le granulé (29) de matière plastique est rassemblé encore en emballages (37) de type en sac, après l'ouverture desquels l'envoi du granulat (22) au dispositif (19) d'extrusion a lieu dans le cadre de la première chaîne de fabrication, ou
- lorsque l'on envoie le granulé (29) de matière plastique, dans le cadre d'une individualisation dans la première chaîne de fabrication dans le cadre d'un envoi (27) en nappe de type en vrac ou par trémie à la vis (21) du dispositif (29) d'extrusion.

2. Procédé suivant la revendication 1, **caractérisé en ce que** l'on applique, à la matière plastique, notamment sous forme de granulé, le rayonnement riche en énergie sous la forme
- d'un rayonnement beta,
- d'un rayonnement gamma,
- d'un rayonnement UV,
- d'un rayonnement UV monochromatique,
- d'un rayonnement à rayon X, ou
- d'une suite de courts éclairs lumineux.

3. Procédé suivant la revendication 1 ou 2, **caractérisé en ce que**, pour le traitement au plasma de la matière plastique, notamment sous forme de granulés, on utilise
- un plasma atmosphérique.

4. Procédé suivant l'une des revendications précédentes, **caractérisé en ce que** l'on utilise, pour la matière plastique, notamment sous forme de granulés, comme gaz à effet stérilisant
- de l'ozone,
- du peroxyde d'hydrogène,
- de l'oxyde d'éthylène ou
- du dioxyde d'azote.

5. Procédé suivant l'une des revendications précédentes, **caractérisé en ce que** le stade de traitement s'effectue en traitant le granulé (29) de matière plastique, individualisé par déplacement, au moins le long d'une partie de sa surface avec une profondeur de pénétration petite, de préférence de quelques microns.

6. Procédé suivant l'une des revendications précédentes, **caractérisé en ce que** l'on utilise, comme matière plastique à traiter, des thermoplastiques, notamment des polymères, comme PE, HDPE, PP, PET, COC, COP, EVOH et PA, de préférence des matériaux faciles à extruder, comme du polyéthylène basse densité (LDPE), du PP ou de l'HDPE.

7. Procédé suivant l'une des revendications précédentes, **caractérisé en ce que** l'on effectue les divers stades de fabrication d'une manière stérile à des cadences de fabrication pour un produit (11) du type contenant ayant peu de germes rempli et fermé, de 2 à 4 secondes.

8. Procédé suivant l'une des revendications précédentes, **caractérisé en ce que** l'on effectue, au moyen du dispositif (19) d'extrusion, un dispositif de co-extrusion, ce qui donne une structure stratifiée de la paroi du produit (11) du type contenant fermé et rempli, et **en ce que** l'on ne soumet à l'un des stades de traitement précédents de réduction de polluants biologiques que le polymère formant la paroi intérieure du récipient (11) ainsi co-extrudé.
